# EUROPÄISCHE PATENTSCHRIFT

(11) **EP 3 212 099 B1**
(45) Veröffentlichungstag und Bekanntmachung des Hinweises auf die Patenterteilung: **07.08.2019**
(21) Anmeldenummer: 15790442.6
(22) Anmeldetag: 22.10.2015
(51) Int. Cl.: A61B 17/88

(54) **VORRICHTUNG ZUM APPLIZIEREN VON KNOCHENERSATZPASTEN**
DEVICE FOR APPLYING BONE REPLACEMENT PASTES
DISPOSITIF POUR APPLIQUER DES SUBSTITUTS OSSEUX SOUS FORME DE PÂTE

(30) Priorität: 27.10.2014 DE 202014008486 U
(43) Veröffentlichungstag der Anmeldung: 06.09.2017
(73) Patentinhaber: Mathys AG Bettlach, 2544 Bettlach (CH)
(72) Erfinder: HUG, Yvonne, 4800 Zofingen (CH); OLBRICHT, Björn, 2503 Biel (CH)
(74) Vertreter: Lusuardi, Werther
(86) Internationale Anmeldenummer: PCT/CH2015/000155
(87) Internationale Veröffentlichungsnummer: WO 2016/065490

(56) Entgegenhaltungen:
- EP-A1- 2 517 662
- WO-A1-2007/122006
- WO-A1-2013/013081
- DE-B- 1 278 324
- DE-U1- 20 219 270
- US-A1- 2005 228 397
- US-A1- 2007 027 230
- US-A1- 2012 008 455

## Beschreibung

Die Erfindung bezieht sich auf eine Vorrichtung zum Applizieren von Knochenersatzpasten oder Knochenzementen, einen Kit zum Applizieren von Knochenersatzpasten oder Knochenzementen und ein Verfahren für die intra-operative Zubereitung einer Knochenersatzpaste und Befüllung von Appliziervorrichtungen.

Vertebroplastie ist eine wirksame Behandlungsoption insbesondere für osteoporotische Wirbelfrakturen und für die Augmentation von Wirbelkörpern. Verschiedene injizierbare Biomaterialien wie Polymethylmethacrylat (PMMA) - ein autopolymerisierendes ZweiKomponenten-System - oder Calcium-Phosphat Zemente - durch Einwirkung von Wasser hydraulisch härtende Zemente - sind bekannt und werden zusammen mit geeigneten Zement-Injektionsinstrumenten verwendet.

Neben Knochenzementen werden auch nicht-aushärtende, nicht-polymerisierende oder nicht-hydraulisch härtende Knochenersatzpasten in stabilisierten kyphoplastischen Defekten und Fusionen der Zwischenwirbelbereiche in und um die Zwischenwirbelimplantate (Interbody Fusion) angewendet. Generell werden krankheits-, unfall- oder operationsbedingte Knochendefekte mit verschiedenen Knochenersatzmaterialien behandelt. Neben autologem Knochen bietet synthetisches Knochenersatzmaterial mehrere Vorteile im Vergleich zu allogenem oder xenogenem Knochenersatzmaterial: keine Gefahr einer Übertragung von Infektionserregern, konstante Produkteigenschaften, keine ethischen Bedenken. Um die Applikation von synthetischen Knochenersatzmaterialien in verschiedenen Darreichungsformen wie Granulate, Pasten und Zementen zu erleichtern oder minimal invasiv zu applizieren ist die Verwendung von Dosier-/Appliziersystemen mit einer oder mehreren Appliziervorrichtungen erforderlich.

Zur Ausführung von solchen knochenersatz-erfordernden Behandlungen (Kypoplastie, Vertebroplastie, Interbody Fusion, generell Knochendefekt-Füllungen in der Wirbelsäulenbehandlung oder Orthopädie, Trauma-, CMF- und Dentalbehandlungen) werden eine Mischvorrichtung zum Mischen der Komponenten und mindestens eine Appliziervorrichtung zum Applizieren der Knochenersatzpaste durch eine Kanüle benötigt.

Eine Dosiervorrichtung zum Befüllen von mehreren Appliziervorrichtungen mit einem frisch gemischten Knochenzement ist aus dem Dokument WO 2005/123162 bekannt. Diese bekannte Dosiervorrichtung umfasst eine Dosiereinrichtung in Form einer grossvolumigen Spritze und eine Verteilvorrichtung, welche eine mit der Dosiervorrichtung verbindbare Eintrittsöffnung und daran anschliessend einen Rohrzylinder mit mehreren seitlichen Rohrstutzen umfasst, wobei jeder Rohrstutzen mit einer Spritze verbindbar ist, so dass die an den Rohrstutzen angebrachten Spritzen im Wesentlichen gleichzeitig mit einem Knochenzement befüllbar sind. Jeder Rohrstutzen ist mittels eines Ventils mit dem Rohrzylinder verbunden, so dass die an der Verteilvorrichtung befestigten Spritzen aufeinanderfolgend oder auch im Wesentlichen gleichzeitig gefüllt werden können. Diese bekannte Dosiervorrichtung weist jedoch den Nachteil auf, dass der Benutzer beim Befüllen der Spritzen eine grosse Druckkraft manuell auf den Kolben der Dosiervorrichtung ausüben muss, weil der Rohrzylinder der Verteilvorrichtung mit den Rohrstutzen für die Spritzen relativ lang ist, was beim Befüllen der Spritzen mit hochviskosen Knochenzement einen hohen Druckverlust verursacht und zusätzlich der Übergang zwischen der Kammer der Dosiereinrichtung und der Eintrittsöffnung der Verteilvorrichtung eine relativ starke Querschnittsverengung bildet, welche wiederum einen hohen Druckverlust verursacht.

Aus der US-A 2013/064033 LIDGREN ET AL. ist eine weitere Dosiervorrichtung zum gleichzeitigen Befüllen von mehreren Spritzen bekannt. Diese bekannte Dosiervorrichtung umfasst ebenfalls eine Dosiereinrichtung in Form einer grossvolumigen Spritze und eine an die Austrittsöffnung der Spritze anschliessbare Verteilvorrichtung, welche einen Eintrittsstutzen mit einer Kammer und mehrere sternförmig an der Kammer angeordnete Austrittsstutzen umfasst. Die Austrittsstutzen sind mit je einer Spritze verbindbar. Diese bekannte Dosiervorrichtung weist ebenfalls den Nachteil auf, dass der Benutzer beim Befüllen der Spritzen eine grosse Druckkraft manuell auf den Kolben der Dosiereinrichtung ausüben muss. Dies insbesondere weil der Übergang vom Hohlraum der Spritze zur Austrittsöffnung eine starke Einschnürung bildet, welche insbesondere bei hochviskosen Substanzen einen hohen Druckverlust verursacht und zudem in der Verteilvorrichtung durch die Umlenkung des Knochenzements ein weiterer erheblicher Druckverlust entsteht.

Ferner offenbart US2005/0228397 A1 eine Vorrichtung zum Applizieren von Knochenersatzpasten oder Knochenzementen, wobei die Vorrichtung umfasst:
A) ein gefässartiges Bodenteil mit einem ersten Ende, einem zweiten Ende und einer Kavität, welche eine erste Längsachse definiert und am zweiten Ende offen ist; und
B) einen gleitbar in die Kavität einführbaren Füllkolben mit einer zweiten Längsachse und zwei oder mehreren Hohlräumen, welche den Füllkolben koaxial oder parallel zur zweiten Längsachse durchdringen und in welche wahlweise eine Kanüle einer Appliziervorrichtung einführbar ist.

Hier will die Erfindung Abhilfe schaffen. Der Erfindung liegt die Aufgabe zugrunde, eine Vorrichtung zu schaffen, welche ein Befüllen von Appliziervorrichtungen mit hochviskosen Substanzen mit einem reduzierten Kraftaufwand ermöglicht.

Die Erfindung löst die gestellte Aufgabe mit einer Vorrichtung zum Applizieren von Knochenersatzpasten oder Knochenzementen, welche die Merkmale des Anspruchs 1 aufweist, mit einem Kit zum Applizieren von Knochenersatzpasten oder Knochenzementen, welcher die Merkmale des Anspruchs 11 aufweist und mit einem Verfahren für die intra-operative Zubereitung einer Knochenersatzpaste und Befüllung von Appliziervorrichtungen, welches die Merkmale des Anspruchs 14 aufweist.

Die durch die Erfindung erreichten Vorteile sind im Wesentlichen darin zu sehen, dass dank der erfindungsgemässen Vorrichtung:
- durch die parallele Anordnung der Hohlräume im Füllkolben ein grosses Verhältnis zwischen der Summe der Querschnittsflächen der Hohlräume im Füllkolben und der Querschnittsfläche der Kavität im Bodenteil erreichbar ist, so dass die Fliessgeschwindigkeit der hochviskosen Knochenersatzpaste in den Hohlräumen des Füllkolbens bei dessen Einführung in die mit Knochenersatzpaste gefüllte Kavität im Bodenteil niedrig bleibt, was zu einem reduzierten Druckverlust infolge der Wandreibung der Knochenersatzpaste führt;
- aufgrund dieses grossen Verhältnisses zwischen der Summe der Querschnittsflächen der Hohlräume im Füllkolben und der Querschnittsfläche der Kavität im Bodenteil der durch die Querschnittsreduktion verursachte Druckverlust bei der Einführung des Füllkolbens in die mit Knochenersatzpaste gefüllte Kavität im Bodenteil reduziert wird, so dass die vom Benutzer aufzuwendende Einspritzkraft weiter reduziert wird;
- aufgrund der reduzierten Fliessgeschwindigkeit der Knochenersatzpaste eine Beschädigung der in der Knochenersatzpaste enthaltenen Partikel durch die Wandreibung verhindert werden kann,
- beim Befüllen der Kanülen der Appliziervorrichtungen mit hochviskosen Substanzen infolge der direkten Einführung der Kanülen in die Hohlräume im Füllkolben nur kurze Fliesswege auftreten und somit geringere Druckverluste zu überwinden sind, so dass für den Benutzer ein reduzierter manueller Kraftaufwand resultiert; und
- durch das gleichzeitige Zurückschieben der Applizierkolben beim Befüllen der Appliziervorrichtungen ein initiales Verklemmen beim Einführen der Applizierkolben nach dem Befüllen der Appliziervorrichtungen vermieden werden kann.

Eine Komponente einer bevorzugten Knochenersatzpaste kann als trockene Pulvermischung bestehend aus beta TCP-Granulat und Hyaluronsäure-Granulat zur Verfügung gestellt werden. Wird die Pulvermischung mit einer Flüssigkeit, z.B. einer physiologischen Kochsalzlösung, Blut oder dessen Derivate oder Antibiotika angemischt, so quellen die Hyaluronsäure-Granulate an und umschliessen die beta TCP-Granulate. Es entsteht eine nicht-aushärtbare, knetbare Paste, welche für die minimal invasive Applikation von Knochenersatz verwendbar ist.
Der Kit zum Applizieren kann z.B. ebenfalls für andere Knochenersatzpasten, demineralisierte Knochenmatrizen z.B. DBX oder andere Ready-to-use Knochenersatzpasten angewendet werden, welche ohne Applizierhilfe geliefert werden.

Weitere vorteilhafte Ausgestaltungen der Erfindung können wie folgt kommentiert werden:
In einer speziellen Ausführungsform umfasst die Vorrichtung zusätzlich eine oder mehrere Appliziervorrichtungen, welche je eine Kanüle mit einem Lumen und einen in das Lumen der Kanüle einführbaren Applizierkolben umfassen.

In einer anderen Ausführungsform der Vorrichtung weisen die Kavität eine zur ersten Längsachse senkrechte Querschnittsfläche Q und jeder Hohlraum eine zur zweiten Längsachse senkrechte Querschnittsfläche q auf, wobei das Verhältnis zwischen der Summe der Querschnittsflächen q und der Querschnittsfläche Q zwischen 0,25 und 0,95 beträgt, vorzugsweise zwischen 0,35 und 0,9. Aufgrund dieses grossen Verhältnisses zwischen der Summe der Querschnittsflächen q der Hohlräume und der Querschnittsfläche Q der Kavität im Bodenteil wird der durch die Querschnittsänderung verursachte Druckverlust reduziert, so dass die vom Benutzer aufzuwendende Einspritzkraft erheblich reduziert wird. Ferner wird erreicht, dass die Fliessgeschwindigkeit der Knochenersatzpaste in den Hohlräumen niedrig gehalten werden kann, wodurch ein reduzierter Druckverlust infolge der Wandreibung der Knochenersatzpaste erreichbar ist.

In wiederum einer anderen Ausführungsform der Vorrichtung weisen die Kavität senkrecht zur ersten Längsachse und der Füllkolben aussen senkrecht zur zweiten Längsachse eine nicht-kreisförmige Querschnittsfläche auf, vorzugsweise eine kleeblattartige, ovale oder elliptische Querschnittsfläche. Damit ist der Vorteil erreichbar, dass das Verhältnis zwischen der Summe der Querschnittsflächen q im Fall von zwei oder drei Hohlräumen und der Querschnittsfläche Q der Kavität erhöht werden kann, so dass der Druckverlust beim Einführen des Füllkolbens in die Kavität weiter reduziert werden kann.

In einer anderen Ausführungsform der Vorrichtung umfasst der Füllkolben einen Längsabschnitt mit einer verringerten Querschnittsfläche. Durch die verringerte Querschnittsfläche werden Freistellungen zur Reduktion der Reibung erreicht, welche insbesondere beim Einführen des Füllkolbens in die mit Knochenersatzpaste gefüllte Kavität im Bodenteil zur Verringerung des Kraftaufwands führt.

In einer anderen Ausführungsform weist die Kanüle ein Lumen auf, welches am vorderen Ende der Kanüle keine Verengungen umfasst, wobei vorzugsweise das Lumen im Wesentlichen einem Hohlraum des Füllkolbens entspricht.

In einer weiteren Ausführungsform der Vorrichtung ist die Kanüle vollständig oder nur am vorderen Ende röntgenstrahlenopak. Dadurch ist der Vorteil erreichbar, dass bei einer Applikation der Knochenersatzpaste die Position der Kanüle in einer Röntgenaufnahme sichtbar ist und die Knochenersatzpaste sicher und effektiv appliziert werden kann. Als strahlenundurchlässige Materialien (Radio-opacifier) werden oft beispielsweise BaSO₄, dichte keramische oder metallische Stoffe verwendet. Beispielsweise kann das BaSO₄ direkt zu dem Rohmaterial (z.B. Polymer) der Kanüle beigemischt und anschliessend extrudiert werden.

In einer anderen Ausführungsform der Vorrichtung umfasst das trichterartig ausgebildete zweite Ende des Bodenteils innen mindestens eine Rippe. Durch die Rippe kann ein falsches Einführen oder Verklemmen des Füllkolbens verhindert werden.

In einer weiteren Ausführungsform der Vorrichtung umfasst das Bodenteil zusätzlich eine äussere Hülse, welche mit dem zweiten Ende des Bodenteils durch eine flanschartige Erweiterung des Bodenteils verbunden ist und welche sich in Richtung der ersten Längsachse über das erste Ende des Bodenteils hinaus bis zu einem unteren Ende der Hülse ausdehnt.

In einer weiteren Ausführungsform der Vorrichtung umfasst das erste Ende des Bodenteils im Bereich der Längsachse ein kleines Entlüftungsloch.

Der erfindungsgemässe Kit zur Applikation von Knochenersatzpasten umfasst eine Vorrichtung gemäss einer der oben beschriebenen Ausführungsformen und zusätzlich mindestens eine weitere Appliziervorrichtung. Durch diese weitere Appliziervorrichtung ergibt sich der Vorteil, dass die zweite oder die weiteren Appliziervorrichtungen z.B. bereits während der intraoperativen Verwendung der ersten Appliziervorrichtung mit Knochenersatzpaste befüllt werden können.

In einer speziellen Ausführungsform umfasst der Kit zusätzlich mindestens einen weiteren Füllkolben. Dadurch können unterschiedlich ausgebildete Füllkolben eingesetzt werden, z.B. Füllkolben mit jeweils anderen Durchmessern der Hohlräume, so dass auch Kanülen mit anderen Durchmessern eingesetzt werden können.

In einer anderen Ausführungsform des Kits:
a) sind die Hohlräume eines ersten Füllkolbens gegenüber den Hohlräumen eines zweiten Füllkolbens unterschiedlich ausgebildet;
b) weist die Kanüle einer ersten Appliziervorrichtung eine zu den Hohlräumen des ersten Füllkolbens passende Aussenform auf; und
c) die Kanüle einer zweiten Appliziervorrichtung weist eine zu den Hohlräumen des zweiten Füllkolbens passende Aussenform auf.

In einer speziellen Ausführungsform des Verfahrens wird vor dem Befüllen des Bodenteils der folgende zusätzliche Schritt ausgeführt: Mischen der zur Präparation der Knochenersatzpaste erforderlichen Stoffe. Bei sogenannten Ready-to-use Produkten ohne Applikationshilfe ist dieser Schritt nicht notwendig.

Die Erfindung und Weiterbildungen der Erfindung werden im Folgenden anhand der teilweise schematischen Darstellungen mehrerer Ausführungsbeispiele noch näher erläutert.

Es zeigen:
Fig. 1 eine perspektivische Ansicht einer Ausführungsform der erfindungsgemässen Vorrichtung;
Fig. 2 eine Aufsicht vom vorderen Ende auf den Füllkolben einer weiteren Ausführungsform der erfindungsgemässen Vorrichtung;
Fig. 3 eine Aufsicht vom zweiten Ende auf das Bodenteil der in Fig. 2 dargestellten Ausführungsform der erfindungsgemässen Vorrichtung;
Fig. 4 eine Draufsicht auf das Bodenteil und einen darin eingeführten Füllkolben einer anderen Ausführungsform der erfindungsgemässen Vorrichtung;
Fig. 5 einen Längsschnitt durch das Bodenteil mit eingeführtem Füllkolben gemäss einer weiteren Ausführungsform der erfindungsgemässen Vorrichtung;
Fig. 6 eine perspektivische Ansicht des Bodenteils einer anderen Ausführungsform der erfindungsgemässen Vorrichtung;
Fig. 7 eine Draufsicht auf das Bodenteil mit eingeführtem Spatel gemäss einer weiteren Form der erfindungsgemässen Vorrichtung;
Fig. 8 eine schematische Darstellung des Bodenteils mit teilweise eingeführtem Spatel der in Fig. 7 dargestellten Ausführungsform der erfindungsgemässen Vorrichtung;
Fig. 9 eine Draufsicht auf einen Spatel gemäss einer anderen Ausführungsform der erfindungsgemässen Vorrichtung;
Fig. 10a - 10c je eine Seitenansicht von unterschiedlich ausgebildeten Kanülen der Appliziervorrichtungen einer weiteren Ausführungsform der erfindungsgemässen Vorrichtung;
Fig. 11 eine perspektivische Darstellung der Bodenteils mit eingeführtem Füllkolben gemäss einer anderen Ausführungsform der erfindungsgemässen Vorrichtung;
Fig. 12 einen Längsschnitt durch eine weitere Ausführungsform der erfindungsgemässen Vorrichtung mit in das Bodenteil eingeführtem Füllkolben;
Fig. 13 eine Explosionsdarstellung der in Fig. 12 dargestellte Ausführungsform der erfindungsgemässen Vorrichtung;
Fig. 14 eine Draufsicht auf den Füllkolben der in Fig. 12 dargestellten Ausführungsform der erfindungsgemässen Vorrichtung;
Fig. 15 einen Querschnitt durch den Füllkolben entlang der Linie I - I in Fig. 12; und
Fig. 16 eine Draufsicht auf das Bodenteil der in Fig. 12 dargestellten Ausführungsform der erfindungsgemässen Vorrichtung.

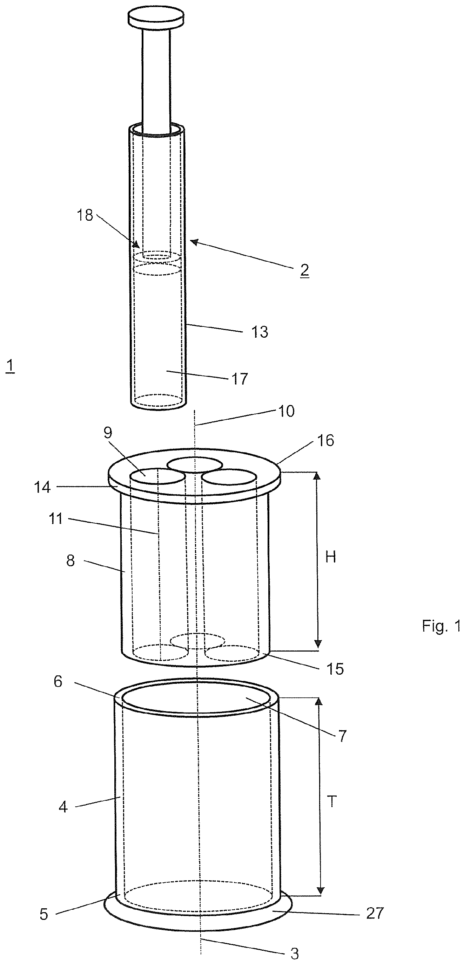

Die in der Fig. 1 dargestellte Ausführungsform der erfindungsgemässen Vorrichtung 1 umfasst ein mit einer Knochenersatzpaste füllbares Bodenteil 4 mit einer Kavität 7, einen in die Kavität 7 einführbaren Füllkolben 8 mit drei Hohlräumen 9 und eine Appliziervorrichtung 2 mit einer wahlweise in einen der drei Hohlräume 9 einführbaren Kanüle 13. Das Bodenteil 4 ist gefässartig ausgebildet und umfasst ein erstes Ende 5, ein zweites Ende 6 und eine Kavität 7, welche eine erste Längsachse 3 definiert. Die Kavität 7 ist am ersten Ende 5 der Bodenteils 4 geschlossen und am zweiten Ende 6 offen. Der gleitbar in die Kavität 7 einführbare Füllkolben 8 umfasst eine zweite Längsachse 10 und beispielhaft drei kreiszylindrische Hohlräume 9, welche den Füllkolben 8 parallel zur zweiten Längsachse 10 durchdringen. Die Hohlräume 9 weisen zur zweiten Längsachse 10 parallele Hohlraumachsen 11 auf, welche in den Ecken eines zur zweiten Längsachse 10 senkrecht stehenden gleichseitigen Dreiecks angeordnet sind. Die Kavität 7 im Bodenteil 4 ist kreiszylindrisch ausgebildet und der Füllkolben 8 ist aussen dazu passend ebenfalls kreiszylindrisch ausgebildet. Der Füllkolben 8 ist gleitbar in die Kavität 7 im Bodenteil 4 einführbar und weist ein geringes Spiel in der Kavität 7 auf, welches dazu geeignet ist, den Widerstand beim Einführen des Füllkolbens 8 zu reduzieren ohne jedoch ein Eindringen von Knochenersatzpaste in den durch das Spiel verursachten Zwischenraum zwischen der Aussenwand des Füllkolbens 8 und der Innenwand der Kavität 7 zu gestatten. Alternativ können die Kavität 7 und der Füllkolben 8 prismatisch ausgebildet sein. Zudem können in einer alternativen Ausführungsform die Hohlräume 9 im Füllkolben 8 prismatisch ausgebildet sein und beispielsweise einen polygonförmigen (z.B. dreieckförmigen, quadratischen, trapezförmigen oder rhomboidförmigen) Querschnitt aufweisen. Die Kavität 7 weist in Richtung der ersten Längsachse 3 gemessen eine Tiefe T auf, während und der Füllkolben 8 in Richtung der zweiten Längsachse 10 gemessen eine Höhe H aufweist, wobei die Höhe H grösser als die Tiefe T ist. Am ersten Ende 5 des Bodenteils 4 ist ein Fuss 27 angeordnet, welcher senkrecht zur ersten Längsachse 3 eine grössere Querschnittsfläche als der oben anschliessende Abschnitt des Bodenteils 4 aufweist und eine Auflagefläche definiert. Ferner ist am hinteren Ende 16 des Füllkolbens 8 ein Griffteil 14 angeordnet. Die Appliziervorrichtung 2 umfasst eine Kanüle 13 mit einem Lumen 17 und einen in das Lumen 13 der Kanüle 13 einführbaren Applizierkolben 18. Die Kanüle 13 kann wahlweise gleitbar in einen der drei Hohlräume eingeführt werden.

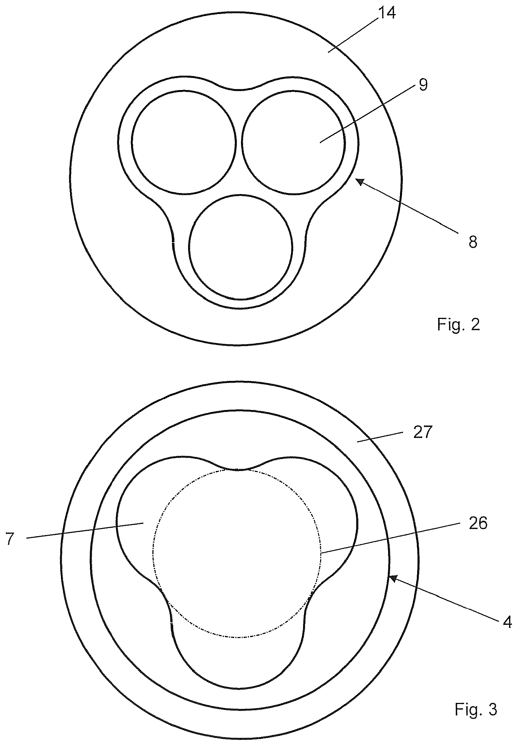

In den Fig. 2 und 3 ist eine weitere Ausführungsform der erfindungsgemässen Vorrichtung 1 dargestellt, deren Bodenteil 4 und Füllkolben 8 sich darin von der in Fig. 1 dargestellten Ausführungsform unterscheiden, dass die Kavität 7 im Bodenteil 4 in einer zur ersten Längsachse 3 senkrechten Querschnittsfläche kleeblattförmig ausgebildet ist und der Füllkolben 8 aussen dazu passend in einer zur zweiten Längsachse 10 senkrechten Querschnittsfläche ebenfalls kleeblattförmig ausgebildet ist. Der Füllkolben 8 ist gleitbar in die Kavität 7 im Bodenteil 4 einführbar und weist ebenfalls ein geringes Spiel in der Kavität 7 auf, welches dazu geeignet ist, den Widerstand beim Einführen des Füllkolbens 8 zu reduzieren ohne jedoch ein Eindringen von Knochenersatzpaste in den durch das Spiel verursachten Zwischenraum zwischen der Aussenwand des Füllkolbens 8 und der Innenwand der Kavität zu gestatten.

Der Füllkolben 8 umfasst ebenfalls drei kreiszylindrische Hohlräume 9, welche den Füllkolben 8 parallel zur zweiten Längsachse 10 durchdringen. Die Hohlräume 9 weisen zur zweiten Längsachse 10 parallele Hohlraumachsen 11 auf, welche in den Ecken eines zur zweiten Längsachse 10 senkrecht stehenden gleichseitigen Dreiecks angeordnet sind. Die Aussenform des Füllkolbens 8 ist derart ausgebildet, dass um je einen Hohlraum 9 - im Querschnitt betrachtet - ein Lappen mit einer kreisbogenförmigen Peripherie gebildet wird, so dass die Hohlraumachse 11 je eines kreiszylindrischen Hohlraums 9 im Zentrum der kreisbogenförmigen Peripherie eines Lappens angeordnet ist. Dabei sind die Durchmesser der kreisbogenförmigen Peripherie der Lappen und der Hohlräume so gewählt, dass die Wandstärken der Aussen- und Zwischenwände eine ausreichende Stabilität der Vorrichtung 1 gewährleisten. Wie in Fig. 3 ersichtlich sind die drei Lappen um eine zentrale Kreisfläche 26 angeordnet und gleichmässig auf dem Umfang dieser zentralen Kreisfläche 26 verteilt. Die Übergänge zwischen den Lappen und der zentralen Kreisfläche 26 sind in einer zur ersten Längsachse 3 senkrechten Querschnittsfläche gerundet, so dass parallel zur ersten Längsachse 3 Verengungen in der Kavität 7 gebildet werden. Passend dazu sind die Übergänge zwischen den Lappen am Füllkolben 8 gerundet, so dass parallel zur zweiten Längsachse 10 konkave Kehlen am Füllkolben 8 gebildet werden. Durch diese Ausbildung der Querschnittsflächen der Kavität 7 und des Füllkolbens 8 wird das Verhältnis zwischen der Summe aller Querschnittsflächen der Hohlräume 9 und der Querschnittsfläche der Kavität 7 vergrössert, so dass auch der Druckverlust, welcher beim Eindrücken des Füllkolbens 8 in die mit Knochenersatzpaste gefüllte Kavität 7 durch die Querschnittsänderung zwischen der Kavität 7 und den Hohlräumen 9 gegenüber der in Fig. 1 dargestellten Ausführungsform mit kreisförmigen Querschnittsflächen der Kavität 7 und Füllkolben 8 erheblich reduziert wird.

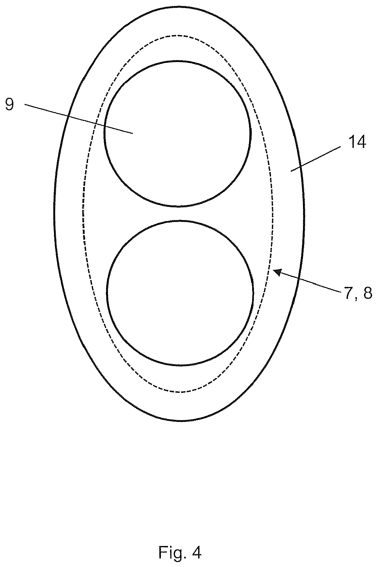

In Fig. 4 ist eine weitere Ausführungsform der erfindungsgemässen Vorrichtung 1 (ohne Appliziervorrichtung 2) dargestellt, welche sich von der in den Fig. 1 und 2 dargestellten Ausführungsform darin unterscheidet, dass die Kavität 7 im Bodenteil 4 und der Füllkolben 8 senkrecht zur ersten, respektive zweiten Längsachse 3;10 eine elliptische Querschnittsfläche aufweisen und dass im Füllkolben 8 zwei Hohlräume 9 angeordnet sind, wobei die zur zweiten Längsachse 10 senkrechte Verbindungslinie zwischen den Hohlraumachsen 11 auf der langen Achse der elliptischen Querschnittsflächen des Füllkolbens 8 und der Kavität 7 liegt. Alternativ können die zur ersten, respektive zweiten Längsachse 3;10 senkrechten Querschnittsflächen der Kavität 7 und des Füllkolbens 8 oval ausgebildet sein.

Nachfolgend sind beispielhafte Abmessungen der Kavität 7 im Bodenteil 4 und der Hohlräume 9 im Füllkolben 8 für zwei unterschiedliche Ausführungsformen der erfindungsgemässen Vorrichtung 1 aufgeführt:

### Erste beispielhafte Ausführungsform:

Die Vorrichtung 1 umfasst eine kreiszylindrisch ausgebildete Kavität 7 im Bodenteil 4 und drei Hohlräume 9 im Füllkolben 8 (Fig. 1). Die Hohlräume 9 im Füllkolben 8 weisen je einen Durchmesser von 5,3 mm und somit je eine Querschnittsfläche q von 22,1 mm² auf, während die Kavität 7 im Bodenteil 4 einen Durchmesser von 14 mm und somit eine Querschnittsfläche Q von 154 mm² aufweisen kann. Für ein Volumen V der Kavität 7 von 10 cm³ weist die Kavität 7 eine Tiefe T von mindestens 65 mm auf. Für ein Volumen v eines Hohlraums 9 von 3,33 cm³ ergibt sich eine Höhe H des Füllkolbens 8 von mindestens 150,7 mm. Das Verhältnis zwischen dem Volumen v eines Hohlraums 9 und dem Volumen V der Kavität 7 beträgt dann 0,33. Das Verhältnis zwischen der Summe der Querschnittsflächen q der Hohlräume 9 und der Querschnittsfläche Q der Kavität 7 beträgt in diesem Fall 0,43.

Der Durchmesser des Lumens 17 in der Kanüle 13 der Appliziervorrichtung 2 und somit auch die Mindestlänge der Kanüle 13 hängt von der Wandstärke der Kanüle 13 ab. Weitere beispielhafte Abmessungen sind:
- die Hohlräume 9 können Längen zwischen 50 und 150 mm für Durchmesser zwischen 3 und 10 mm und Längen zwischen 50 und 100 mm für Durchmesser zwischen 4 und 10 mm aufweisen;
- die Kavität 7 kann Längen zwischen 30 und 100 mm für Durchmesser zwischen 5 und 50 mm und Längen zwischen 40 und 80 mm für Durchmesser zwischen 10 und 20 mm aufweisen; und
- die Kanülen 13 können Längen zwischen 50 und 300 mm für Lumendurchmesser zwischen 3 und 10 mm und Längen zwischen 100 und 200 mm für Lumendurchmesser zwischen 4 und 10 mm aufweisen.

Typischerweise weisen das Lumen 17 einen Durchmesser von 5,2 mm und die Kanüle 13 eine Länge von 200 mm auf.

### Zweite beispielhafte Ausführungsform:

Eine dichteste Anordnung der Hohlräume 9 im Füllkolben 8 ist mit 7 Hohlräumen 9 erreichbar, wobei ein zentraler Hohlraum 9 von 6 weiteren Hohlräumen 9 umgeben wird, deren Hohlraumachsen 11 - in einer zur zweiten Längsachse 10 senkrechten Querschnittsfläche des Füllkolbens 8 - in den Ecken eines Sechsecks angeordnet sind, wobei der Durchmesser der Kavität 7 wenig mehr als das Dreifache des Durchmessers eines Hohlraums 9 beträgt. Die Hohlräume 9 im Füllkolben 8 weisen je einen Durchmesser von 8 mm und somit je eine Querschnittsfläche q von 50,25 mm² auf, während die Kavität 7 im Bodenteil 4 einen Durchmesser von 26 mm und somit eine Querschnittsfläche Q von 531 mm² aufweisen kann. Für ein Volumen V der Kavität 7 von 24,5 cm³ weist die Kavität 7 eine Tiefe T von mindestens 46 mm auf. Für ein Volumen v eines Hohlraums 9 von 3,5 cm³ ergibt sich eine Höhe H des Füllkolbens 8 von mindestens 70 mm. Das Verhältnis zwischen dem Volumen v eines Hohlraums 9 und dem Volumen V der Kavität 7 beträgt dann 0,14. Im Falle einer solchen dichtesten Anordnung ergibt sich ein Verhältnis zwischen der Summe der Querschnittsflächen q der Hohlräume 9 und der Querschnittsfläche Q der Kavität 7 von 0,66.

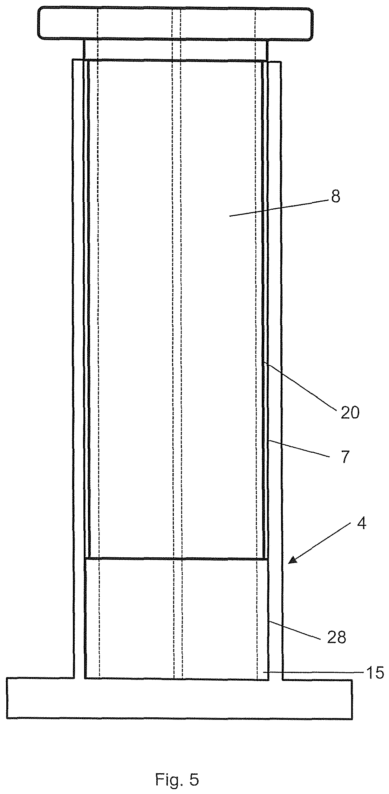

In Fig. 5 ist eine Ausführungsform des Füllkolbens 8 dargestellt, welche sich dadurch auszeichnet, dass der Füllkolben 8 am vorderen Ende 15 eine ersten Längsabschnitt 28 mit einer zur Querschnittsfläche der Kavität 7 passenden Querschnittsfläche (wie oben beschrieben) und an diesen ersten Längsabschnitt 28 anschliessend einen zweiten Längsabschnitt 20 mit einer verringerten Querschnittsfläche umfasst, so dass beim Einführen des Füllkolbens 8 in die Kavität 7 der Reibungswiderstand reduziert wird.

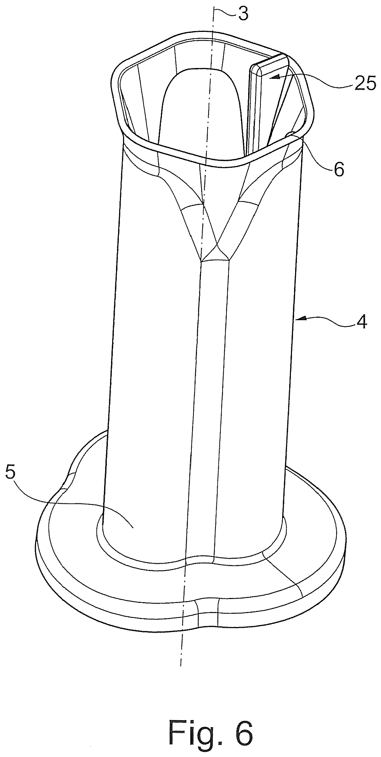

In Fig. 6 ist eine Ausführungsform des Bodenteils 4 dargestellt, welche sich dadurch auszeichnet, dass das zweite Ende 6 des Bodenteil 4 trichterartig ausgebildet ist, wobei sich das trichterartig ausgebildete zweite Ende 6 innen mit einer geringen Steigung gegen die Kavität 7 verjüngt, so dass sich der Füllkolben beim Einführen in die Kavität nicht verklemmen kann und mit einer leichten Drehbewegung in die richtige Position gebracht werden kann. Ferner ist innen am trichterartig ausgebildeten zweite Ende 6 des Bodenteils 4 mindestens eine Rippe 25 angeordnet, welche sich parallel zur ersten Längsachse 3 erstreckt, so dass ein falsches Einführen des Füllkolbens verhindert werden kann.

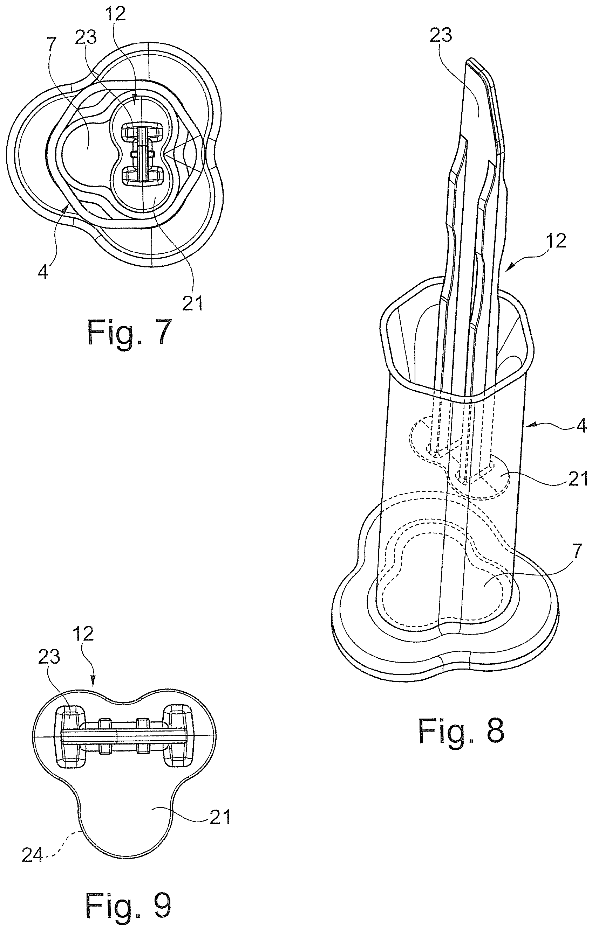

In den Fig. 7 und 8 ist eine Ausführungsform eines mit der erfindungsgemässen Vorrichtung 1 gemäss den Fig. 2 und 3 verwendbaren Spatels 12 dargestellt, welcher zum Befüllen des Bodenteils 4 mit der vorbereiteten Knochenersatzpaste und zum Festdrücken der Knochenersatzpaste in der Kavität 7 des Bodenteils 4 dient. Der Spatel 12 umfasst einen Spatelfuss 21 und einen Stiel 23. Der Spatelfuss 21 überdeckt in dieser Ausführungsform nur einen Teil der Querschnittsfläche der Kavität 7 und hat senkrecht zum Stiel 23 eine Grundfläche, welche im Wesentlichen die Form einer liegenden Acht aufweist. Die beiden Rundungen der Acht sind passend zu zwei aneinandergrenzenden Lappen der kleeblattförmigen Querschnittsfläche der Kavität 7 ausgebildet, so dass der Spatelfuss 21 in einer wählbaren Orientierung in der Kavität 7 geführt bewegbar ist. In der Knochenersatzpaste und im Bodenteil 4 eingeschlossene Luft kann daher beim Festdrücken der Knochenersatzpaste durch einen Kanal entweichen, dessen Querschnitt durch den dritten Lappen der kleeblattförmigen Querschnittsfläche der Kavität 7 gebildet wird.

In Fig. 9 ist eine weitere Ausführungsform eines mit der erfindungsgemässen Vorrichtung 1 gemäss den Fig. 2 und 3 verwendbaren Spatels 12 dargestellt. In der hier dargestellten Ausführungsform weist der Spatelfuss 21 eine Grundfläche 24 auf, welche zu der zur ersten Längsachse 3 des Bodenteils 4 senkrechten Querschnittsfläche der Kavität 7 geometrisch ähnlich ist, so dass der Spatelfuss 21 am Umfang ein Spiel gegenüber der Kavität 7 aufweist. In diesem Fall kann die in der Knochenersatzpaste und im Bodenteil eingeschlossene Luft beim Festdrücken der Knochenersatzpaste durch den Zwischenraum entweichen, welcher durch das Spiel zwischen dem Spatelfuss 21 und der Innenwand der Kavität 7 gebildet wird.

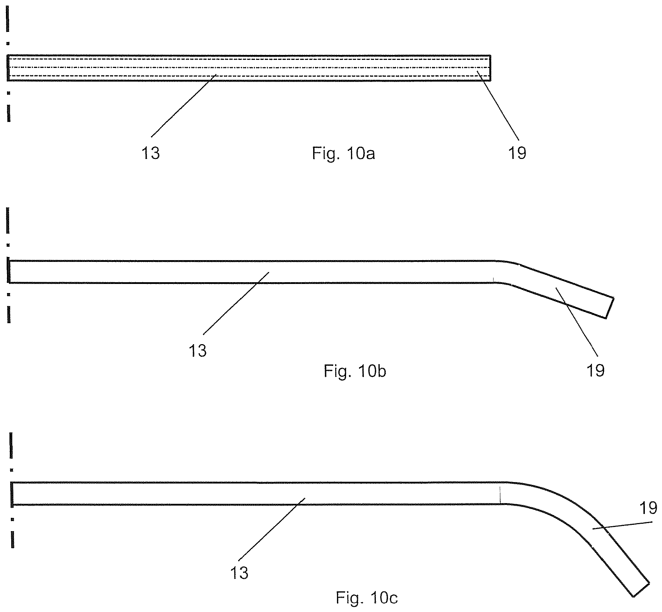

Die Fig. 10a bis 10c zeigen unterschiedliche Ausführungsformen der Kanülen 13, welche:
- als gerade Kanülen 13 ausgebildet sein können (Fig. 10a);
- an dem vorderen Ende 19 der Kanüle 13 mit einem Winkel zwischen 5° und 50°, vorzugsweise zwischen 10° und 45° vorgebogen sein können (Fig. 10b); oder
- am vorderen Ende 19 der Kanüle 13 auf einer Länge zwischen 0 - 70 mm, vorzugsweise 0 - 60 mm biegbar sein können, so dass die Kanülen 13 intraoperativ biegbar sind (Fig. 10c);
- die Kanülen 13 können röntgenstrahlendurchlässig (ohne Radio-opacifier) ausgebildet sein; oder
- die Kanülen 13 können einen Röntgenmarker am vorderen Ende (Kontakt zu Defekt) aufweisen; oder
- die Kanülen 13 können über dem gesamten Kanülenvolumen röntgendicht ausgebildet sein (z.B. durch Beigabe von BaSO₄ an das Rohmaterial der Kanülen).

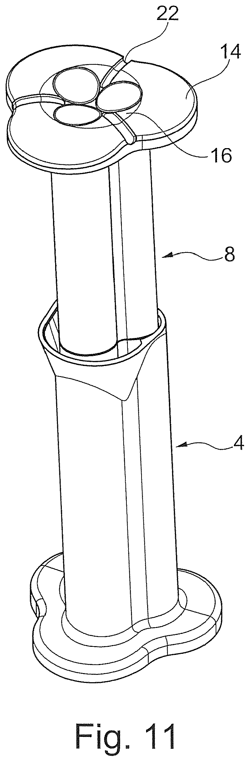

Fig. 11 zeigt eine weitere Ausführungsform der erfindungsgemässen Vorrichtung 1, welche sich von der in den Fig. 2 und 3 dargestellten Ausführungsform nur darin unterscheidet, dass am hinteren Ende 16 des Füllkolbens 8 in der endständigen Oberfläche des Griffteils 14 Lüftungsschlitze 22 angeordnet sind, welche sich von einer zentralen Vertiefung zwischen den Hohlräumen 9 am hinteren Ende 16 des Füllkolbens 8 nach aussen erstrecken, so dass beim Eindrücken des Füllkolbens 8 in die Kavität 7 mittels eines das hintere Ende 16 des Füllkolbens 8 bedeckenden Gegenstands Luft aus den Hohlräumen 9 entweichen kann. Ferner können zwischen den Hohlräumen 9 im Füllkolben 8, in welche die Kanüle 13 der Appliziervorrichtung 2 eingeführt wird, Verbindungen (Löcher oder Rillen, nicht gezeichnet) angeordnet sein, welche für einen Luftausgleich zwischen den Hohlräumen dienen.

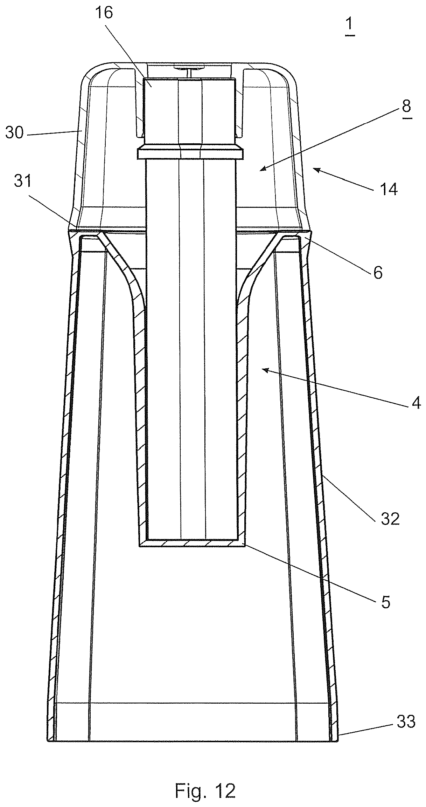

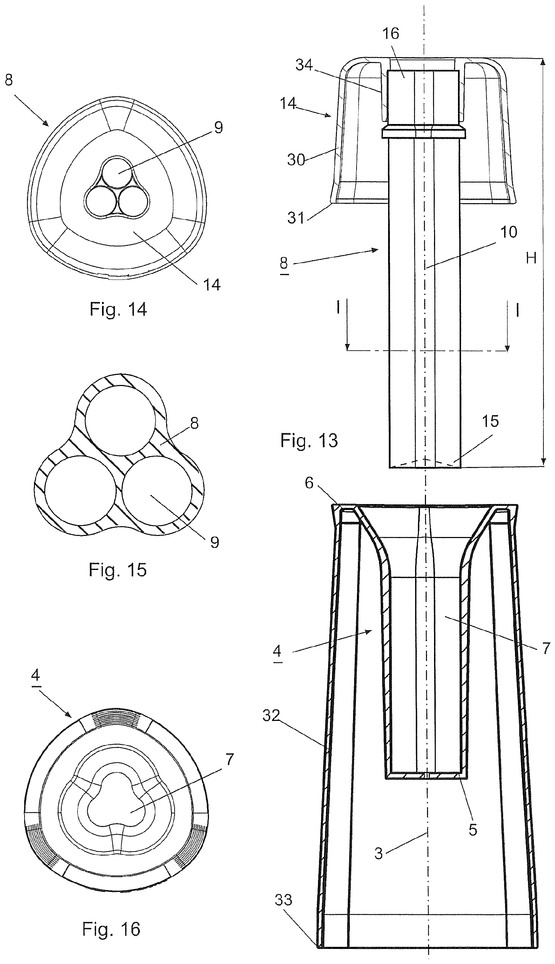

Eine weitere Ausführungsform der erfindungsgemässen Vorrichtung 1 ist in den Fig. 12 bis 16 dargestellt, welche sich von der in den Fig. 2 und 3 dargestellten Ausführungsform nur darin unterscheidet, dass das Bodenteil 4 anstelle des am ersten Ende 5 angeordneten flanschförmigen Fusses 27 mit einer äusseren Hülse 32 ausgestattet ist und der Füllkolben 8 ein haubenförmiges Griffteil 14 umfasst, welches eine sich in Richtung der zweiten Längsachse 10 gegen das vordere Ende 15 auf einem Abschnitt der Höhe H des Füllkolbens 8 ausdehnende Kappe 30 bildet. Die äussere Hülse 32 ist mit dem zweiten Ende 6 des Bodenteils 4 durch eine flanschartige Erweiterung des Bodenteils 4 verbunden und dehnt sich in Richtung der ersten Längsachse 3 über das erste Ende 5 des Bodenteils 4 hinaus bis zum unteren Ende 33 der Hülse 32 aus.

Der Füllkolben 8 ist an seinem hinteren Ende 16 mittels Ultraschall in einer Aufnahme 34 in der Kappe 30 verschweißt. Die Kappe 30 und der Füllkolben 8 bilden für den Anwender eine Einheit. Alternativ kann die Verbindung zwischen dem hintern Ende 16 des Füllkolbens 8 und der Aufnahme 34 in der Kappe 30 auch als irreversible Presssitz-Verbindung oder durch eine irreversible Schnappverbindung ausgeführt sein. In anderen Ausführungsformen können die Kappe 30 und der Füllkolben 8 auch einstückig ausgebildet sein.

Die Kappe 30 und die Hülse 32 sind derart ausgebildet, dass sich die Aussenform der Kappe 30 vom hinteren Ende 16 des Füllkolbens 8 gegen das untere Ende 31 der Kappe 30 erweitert und die Aussenform der Hülse 32 sich vom zweiten Ende 6 des Bodenteils 4 gegen das untere Ende 33 der Hülse 32 erweitert.

Die Kappe 30 und die Hülse 32 haben beispielhaft und nicht einschränkend je eine zur ersten Längsachse 3, respektive zur zweiten Längsachse 10 orthogonale Querschnittsfläche, welche die Form eines ausgebauchten Dreiecks ähnlich einem Reuleaux-Dreieck aufweist und deren Kontur aus drei abflachten Kreisbögen mit abgerundeten Ecken zusammengesetzt ist. Durch diese Formgebung wird erreicht, dass die Kappe 30 und/oder die Hülse 32 einerseits ergonomisch an eine Verwendung von Hand angepasst sind und andererseits nicht davonrollen, wenn eines der Teile oder die zusammengesetzte Vorrichtung 1 auf die Seitenfläche fällt.

Bei vollständig in die Kavität 7 des Bodenteils 4 eingeführtem Füllkolben 8 liegt das untere Ende 31 der Kappe 30 auf dem zweiten Ende 6 des Bodenteils 4 auf. Die Kanten am unteren Ende 31 der Kappe 30 und am zweiten Ende 6 des Bodenteils 4 sind abgerundet, damit der Handschuh des Benutzers nicht verletzt wird. Die Kappe 30 und die Hülse 32 weisen eine leichte Ausweitung auf, um ein Abrutschen der Hände zu verhindern. Die Hülse 32 hat zusätzlich auf der Aussenfläche noch eine dreidimensionale Strukturierung um ein Abrutschen der Handschuhe zu verhindern.

Das vordere Ende 15 des Füllkolbens 8 ist beispielhaft und nicht einschränkend konkav ausgebildet, wobei das konkav ausgebildete vordere Ende 15 des Füllkolbens 8 im Bereich der zweiten Längsachse 10 um 0,4 mm bis 1,2 mm vertieft ist. Alternativ kann das vordere Ende 15 des Füllkolbens 8 auch eben ausgebildet sein. Ferner umfasst das erste Ende 5 des Bodenteils 4 im Bereich der ersten Längsachse 3 ein kleines Entlüftungsloch 29.

Analog zu der in Fig. 4 dargestellten Ausführungsform kann das zweite Ende 6 des Bodenteils 4 trichterartig ausgebildet sein, wobei sich das trichterartig ausgebildete zweite Ende 6 innen mit einer geringen Steigung gegen die Kavität 7 verjüngt, so dass sich der Füllkolben beim Einführen in die Kavität nicht verklemmen kann und mit einer leichten Drehbewegung in die richtige Position gebracht werden kann.

### Intra-operative Zubereitung der Knochenersatzpaste und Befüllung der Appliziervorrichtungen (Fig. 1 und 5 bis 9)

1) Mischen der zur Präparation der Knochenersatzpaste erforderlichen Stoffe.
   Die trockene Pulvermischung wird mit Flüssigkeit unter Verwendung eines Spatels 12 angerührt. Bei Ready-to-use Produkten ohne Applikationshilfe, fällt dieser Schritt weg;
2) Befüllen des Bodenteils 4 mit der vorbereiteten Knochenersatzpaste.
   Die vorbereitete Knochenersatzpaste wird mit Spatel 12 (vorzugsweise über die Trichterfunktion) in das Bodenteil der Ladeeinheit gefüllt;
3) Festdrücken der Knochenersatzpaste in der Kavität 7 des Bodenteils 4.
   Mit dem Spatelfuss 21 kann die Masse in das Bodenteil 4 "heruntergestösselt" werden;
4) Einführen und Eindrücken des Füllkolbens 8 in die Kavität 7 des Bodenteils 4, so dass die Hohlräume 9 des Füllkolbens 8 mit Knochenersatzpaste gefüllt werden;
   Sobald die gesamte Masse in das Bodenteil befüllt wurde, wird der Füllkolben mit den 3 Hohlzylindern von Hand auf das Bodenteil gedrückt. Dadurch werden die Hohlräume des Füllkolbens gefüllt. In diesem Fall wurden die Querschnittsflächen ebenfalls so optimiert, dass ein möglichst geringer Kraftaufwand resultiert.
5) Einführen der Kanüle 13 einer Appliziervorrichtung 2 in einen der mit Knochenersatzpaste gefüllten Hohlräume 9 des Füllkolbens 8, so dass die im Hohlraum 9 des Füllkolbens 8 vorhandene Knochenersatzpaste in das Lumen 17 der Kanüle 13 der Appliziervorrichtung 2 befördert wird.
   Vorzugsweise wird dabei der Füllkolben 8 am Griffelement 14 gegen das Bodenteil 4 gedrückt, so dass der Füllkolben 8 durch den Druck auf die Knochenersatzpaste in der Kavität 7 des Bodenteils 4 nicht angehoben wird. Die Knochenersatzpaste kann durch eine leichte Drehung der Appliziervorrichtung 2 abgestochen werden.
6) Einbringen der Knochenersatzpaste in den Knochendefekt mittels der Appliziervorrichtung 2.

Die Schritte 5) und 6) können nach Bedarf mehrmals wiederholt werden bis der gesamte Knochendefekt mit Knochenersatzpaste gefüllt ist.

Eine Ausführungsform des erfindungsgemässen Kits umfasst neben einer Vorrichtung 1 gemäss einer in den Fig. 1 bis 10 dargestellten Ausführungsformen mindestens eine weitere Appliziervorrichtung 2, wodurch ermöglicht wird, dass z.B. bereits während der intraoperativen Verwendung der ersten Appliziervorrichtung 2 eine oder mehrere weitere Appliziervorrichtungen 2 mit Knochenersatzpaste befüllt werden können. In weiteren Ausführungsformen des erfindungsgemässen Kits kann dieser zusätzlich einen oder mehrere weitere Füllkolben 8 umfassen, so dass unterschiedlich ausgebildete Füllkolben 8 eingesetzt werden können, z.B. Füllkolben 8 mit jeweils anderen Durchmessern der Hohlräume 9, so dass auch Kanülen 13 mit anderen Durchmessern eingesetzt werden können. Alternativ können die Hohlräume 9 eines ersten Füllkolbens 8 gegenüber den Hohlräumen 9 eines zweiten Füllkolbens 8 unterschiedlich ausgebildet sein (z.B. kreiszylindrische Hohlräume 9 gegenüber prismatischen Hohlräumen 9), wobei mindestens die Kanüle 13 einer ersten Appliziervorrichtung 2 eine zu den Hohlräumen 9 des ersten Füllkolbens 8 passende Aussenform aufweist (z.B. kreiszylindrisch) und die Kanüle 13 einer zweiten Appliziervorrichtung 2 eine zu den Hohlräumen 9 des zweiten Füllkolbens 8 passende Aussenform aufweist (z.B. prismatisch).

Obwohl wie oben beschrieben verschiedene Ausführungsformen der vorliegenden Erfindung vorliegen, sind diese so zu verstehen, dass die verschiedenen Merkmale sowohl einzeln als auch in jeder beliebigen Kombination verwendet werden können.

Diese Erfindung ist daher nicht einfach auf die oben erwähnten, besonders bevorzugten Ausführungsformen beschränkt.

## Patentansprüche

1. Vorrichtung (1) zum Applizieren von Knochenersatzpasten oder Knochenzementen, wobei die Vorrichtung (1) umfasst:
A) ein gefässartiges Bodenteil (4) mit einem ersten Ende (5), einem zweiten Ende (6) und einer Kavität (7), welche eine erste Längsachse (3) definiert, am ersten Ende (5) geschlossen ist und am zweiten Ende (6) offen ist; und
B) einen gleitbar in die Kavität (7) einführbaren Füllkolben (8) mit einer zweiten Längsachse (10) und zwei oder mehreren Hohlräumen (9), welche den Füllkolben (8) koaxial oder parallel zur zweiten Längsachse (10) durchdringen und in welche wahlweise eine Kanüle (13) einer Appliziervorrichtung (2) einführbar ist.

2. Vorrichtung nach Anspruch 1, **dadurch gekennzeichnet, dass** die Vorrichtung (1) zusätzlich eine Appliziervorrichtung (2) umfasst, welche eine Kanüle (13) mit einem Lumen (17) und einen in das Lumen (13) der Kanüle (13) einführbaren Applizierkolben (18) umfasst.

3. Vorrichtung nach Anspruch 1 oder 2, **dadurch gekennzeichnet, dass** die Kavität (7) eine zur ersten Längsachse (3) senkrechte Querschnittsfläche Q aufweist, jeder Hohlraum (9) eine zur zweiten Längsachse (10) senkrechte Querschnittsfläche q aufweist, und das Verhältnis zwischen der Summe der Querschnittsflächen q und der Querschnittsfläche Q zwischen 0,25 und 0,95 beträgt, vorzugsweise zwischen 0,35 und 0,9.

4. Vorrichtung nach einem der Ansprüche 1 bis 3, **dadurch gekennzeichnet, dass** die Kavität (7) senkrecht zur ersten Längsachse (3) und der Füllkolben (8) aussen senkrecht zur zweiten Längsachse (10) eine nicht-kreisförmige Querschnittsfläche aufweisen, vorzugsweise eine kleeblattartige, ovale oder elliptische Querschnittsfläche.

5. Vorrichtung nach einem der Ansprüche 1 bis 4, **dadurch gekennzeichnet, dass** der Füllkolben (8) einen Längsabschnitt (20) mit einer verringerten Querschnittsfläche umfasst.

6. Vorrichtung nach einem der Ansprüche 1 bis 5, **dadurch gekennzeichnet, dass** die Kanüle (13) ein Lumen (17) aufweist, welches am vorderen Ende (19) der Kanüle (13) keine Verengungen umfasst, wobei vorzugsweise das Lumen (17) im Wesentlichen einem Hohlraum (9) des Füllkolbens (8) entspricht.

7. Vorrichtung nach einem der Ansprüche 1 bis 6, **dadurch gekennzeichnet, dass** die Kanüle (13) vollständig oder nur am vorderen Ende (19) röntgenstrahlenopak ist.

8. Vorrichtung nach einem der Ansprüche 1 bis 7, **dadurch gekennzeichnet, dass** das trichterartig ausgebildete zweite Ende (6) des Bodenteils (4) innen mindestens eine Rippe (25) umfasst.

9. Vorrichtung nach einem der Ansprüche 1 bis 8, **dadurch gekennzeichnet, dass** das Bodenteil (4) zusätzlich eine äussere Hülse (32) umfasst, welche mit dem zweiten Ende (6) des Bodenteils (4) durch eine flanschartige Erweiterung des Bodenteils (4) verbunden ist und welche sich in Richtung der ersten Längsachse (3) über das erste Ende (5) des Bodenteils (4) hinaus bis zu einem unteren Ende (33) der Hülse (32) ausdehnt.

10. Vorrichtung nach einem der Ansprüche 1 bis 9, **dadurch gekennzeichnet, dass** das erste Ende (5) des Bodenteils (4) im Bereich der Längsachse (3) ein kleines Entlüftungsloch (29) umfasst.

11. Kit zur Applikation von Knochenersatzpasten umfassend eine Vorrichtung nach einem der Ansprüche 1 bis 10 und zusätzlich mindestens eine weitere Appliziervorrichtung (2).

12. Kit nach Anspruch 11, **dadurch gekennzeichnet, dass** der Kit zusätzlich mindestens einen weiteren Füllkolben (8) umfasst.

13. Kit nach Anspruch 12, **dadurch gekennzeichnet, dass**:
a) die Hohlräume (9) eines ersten Füllkolbens (8) gegenüber den Hohlräumen (9) eines zweiten Füllkolbens (8) unterschiedlich ausgebildet sind;
b) die Kanüle (13) einer ersten Appliziervorrichtung (2) eine zu den Hohlräumen (9) des ersten Füllkolbens (8) passende Aussenform aufweist; und
c) die Kanüle (13) einer zweiten Appliziervorrichtung (2) eine zu den Hohlräumen (9) des zweiten Füllkolbens (8) passende Aussenform aufweist.

14. Verfahren für die intra-operative Zubereitung einer Knochenersatzpaste und Befüllung von Appliziervorrichtungen (2) unter Verwendung der Vorrichtung (1) gemäss einem der Ansprüche 1 bis 10 **gekennzeichnet durch** die Schritte:
Befüllen des Bodenteils (4) mit der vorbereiteten Knochenersatzpaste;
Einführen und Eindrücken des Füllkolbens (8) in die Kavität (7 des Bodenteils (4), so dass die Hohlräume (9) des Füllkolbens (8) mit Knochenersatzpaste gefüllt werden; und
Einführen einer Kanüle (13) einer Appliziervorrichtung (2) in einen der mit Knochenersatzpaste gefüllten Hohlräume (9) des Füllkolbens (8), so dass die im Hohlraum (9) des Füllkolbens (8) vorhandene Knochenersatzpaste in das Lumen (17) der Kanüle (13) der Appliziervorrichtung (2) befördert wird.

15. Verfahren nach Anspruch 14, **dadurch gekennzeichnet, dass** vor dem Befüllen des Bodenteils der folgende zusätzliche Schritt ausgeführt wird:
Mischen der zur Präparation der Knochenersatzpaste erforderlichen Stoffe.

## Claims

1. A device (1) for administering bone substitute pastes or bone cements, wherein the device (1) comprises:
A) a vessel-type bottom piece (4) with a first end (5), a second end (6), and a cavity (7), which defines a first longitudinal axis (3), is closed at the first end (5) and is open at the second end (6); and
B) a filling piston (8), slidably insertable into the cavity (7), with a second longitudinal axis (10) and two or more hollow spaces (9), which pervade the filling piston (8) coaxially or parallel with respect to the second longitudinal axis (10) and into which a cannula (13) of an administering device (2) can be optionally inserted.

2. The device according to claim 1, **characterized in that** the device (1) additionally comprises an administering device (2), which comprises a cannula (13) having a lumen (17) and an administering piston (18) insertable into the lumen (17) of the cannula (13).

3. The device according to claim 1 or 2, **characterized in that** the cavity (7) has a cross-sectional area Q perpendicular to the first longitudinal axis (3), each hollow space (9) has a cross-sectional area q perpendicular to the second longitudinal axis (10), and the ratio between the sum of the cross-sectional areas q and the cross-sectional area Q is between 0.25 and 0.95, preferably between 0.35 and 0.9.

4. The device according to any one of claims 1 to 3, **characterized in that** the cavity (7) perpendicular to the first longitudinal axis (3) and the filling piston (8) externally perpendicular to the second longitudinal axis (10) have a non-circular cross-sectional area, preferably a clover leaf-type, oval, or elliptical cross-sectional area.

5. The device according to any one of claims 1 to 4, **characterized in that** the filling piston (8) comprises a longitudinal section (20) with a decreased cross-sectional area.

6. The device according to any one of claims 1 to 5, **characterized in that** the cannula (13) has a lumen (17), which comprises no narrowing at the front end (19) of the cannula (13), wherein preferably the lumen (17) essentially corresponds to a hollow space (9) of the filling piston (8).

7. The device according to any one of claims 1 to 6, **characterized in that** the cannula (13) is completely x-ray opaque or x-ray opaque only at the front end (19).

8. The device according to any one of claims 1 to 7, **characterized in that** the funnel-shaped second end (6) of the bottom piece (4) comprises at least one inner rib (25).

9. The device according to any one of claims 1 to 8, **characterized in that** the bottom piece (4) additionally comprises an outer sleeve (32), which is connected to the second end (6) of the bottom piece (4) by means of a flange-type extension of the bottom piece (4) and which extends in the direction of the first longitudinal axis (3) beyond the first end (5) of the bottom piece (4) up to a bottom end (33) of the sleeve (32).

10. The device according to any one of claims 1 to 9, **characterized in that** the first end (5) of the bottom piece (4) comprises a small ventilation hole (29) in the area of the longitudinal axis (3).

11. A kit for administering bone substitute pastes, comprising a device according to any one of claims 1 to 10 and additionally at least one further administering device (2).

12. The kit according to claim 11, **characterized in that** the kit additionally comprises at least on further filling piston (8).

13. The kit according to claim 12, **characterized in that**:
a) the hollow spaces (9) of a first filling piston (8) are designed differently with respect to the hollow spaces (9) of a second filling piston (8);
b) the cannula (13) of a first administering device (2) has an outer shape matching the hollow spaces (9) of the first filling piston (8); and
c) the canula (13) of a second administering device (2) has an outer shape matching the hollow spaces (9) of the second filling piston (8).

14. A method for the intra-operative preparation of a bone substitute paste and filling of administering devices (2) by using the device (1) according to any one of claims 1 to 10, **characterized by** the steps of:
filling the bottom piece (4) with the prepared bone substitute paste;
inserting and impressing the filling piston (8) into the cavity (7) of the bottom piece (4), so that the hollow spaces (9) of the filling piston (8) are filled with bone substitute paste; and
inserting a cannula (13) of an administering device (2) into one of the hollow spaces (9) of the filling piston (8) filled with bone substitute paste, so that the bone substitute paste present in the hollow space (9) of the filling piston (8) is conveyed into the lumen (17) of the cannula (13) of the administering device (2).

15. The method according to claim 14, **characterized in that** prior to filling of the bottom piece, the following additional step is performed:
mixing the substances required for preparation of the bone substitute paste.

## Revendications

1. Dispositif (1) pour l'application de pâtes de substitut osseux ou de ciments osseux, dans lequel le dispositif (1) comprend :
A) un fond (4) en forme de récipient doté d'une première extrémité (5), d'une deuxième extrémité (6) et d'une cavité (7), laquelle définit un premier axe longitudinal (3), est fermée à la première extrémité (5) et ouverte à la deuxième extrémité (6) ; et
B) un piston de remplissage (8) pouvant être guidé de manière glissante dans la cavité (7), doté d'un deuxième axe longitudinal (10) et de deux ou plus espaces creux (9), lesquels traversent le piston de remplissage de manière coaxiale ou parallèle au deuxième axe longitudinal (10) et à l'intérieur desquels une canule (13) d'un moyen applicateur (2) peut être sélectivement introduite.

2. Dispositif selon la revendication 1, **caractérisé en ce que** le dispositif comprend en outre un moyen applicateur (2), lequel comprend une canule (13) dotée d'une lumière (17) et un piston applicateur (18) pouvant être introduit dans la lumière (13) de la canule (13).

3. Dispositif selon la revendication 1 ou 2, **caractérisé en ce que** la cavité (7) présente une section transversale Q perpendiculaire au premier axe longitudinal (3), chaque espace creux (9) présente une section transversale q perpendiculaire au deuxième axe longitudinal (10), et le rapport entre la somme des sections transversales q et la section transversale Q vaut entre 0,25 et 0,95, de préférence entre 0,35 et 0,9.

4. Dispositif selon l'une des revendications 1 à 3, **caractérisé en ce que** la cavité (7) présente perpendiculairement au premier axe longitudinal (3) une section transversale non-circulaire et le piston de remplissage présente extérieurement et perpendiculairement au deuxième axe longitudinal (10) une section transversale non-circutaire, de préférence une section transversale en forme de trèfle, ovale ou elliptique.

5. Dispositif selon l'une des revendications 1 à 4, **caractérisé en ce que** le piston de remplissage (8) comprend une section longitudinale (20) avec une section transversale réduite.

6. Dispositif selon l'une des revendications 1 à 5, **caractérisé en ce que** la canule (13) présente une lumière (17), laquelle ne comprend pas de rétrécissement à l'extrémité avant (19) de la canule (13), dans lequel la lumière (17) correspond de préférence essentiellement à un espace creux (9) du piston de remplissage (8).

7. Dispositif selon l'une des revendications 1 à 6, **caractérisé en ce que** la canule (13) est opaque aux rayons X, entièrement ou seulement à son extrémité avant (19).

8. Dispositif selon l'une des revendications 1 à 7, **caractérisé en ce que** la deuxième extrémité (6) du fond (4), réalisée en forme d'entonnoir, comprend à l'intérieur au moins une strie (25).

9. Dispositif selon l'une des revendications 1 à 8, **caractérisé en ce que** le fond (4) comprend en outre une gaine extérieure (32), laquelle est raccordée à la deuxième extrémité (6) du fond (4) par un épaulement en forme de bride du fond (4) et laquelle s'étend dans la direction du premier axe longitudinal (3) au-dessus de la première extrémité (5) du fond (4) jusqu'à une extrémité inférieure (33) de la gaine (32).

10. Dispositif selon l'une des revendications 1 à 9, **caractérisé en ce que** la première extrémité (5) du fond (4) comprend dans la zone de l'axe longitudinal (3) un petit trou de ventilation (29).

11. Kit d'application de pâtes de substitut osseux comprenant un dispositif selon l'une des revendications 1 à 10, et en outre au moins un moyen d'application (2) supplémentaire.

12. Kit selon la revendication 11, **caractérisé en ce que** le kit comprend en outre au moins un piston de remplissage (8) supplémentaire.

13. Kit selon la revendication 12, **caractérisé en ce que** :
a) les espaces creux (9) d'un premier piston de remplissage (8) sont réalisés différemment des espaces creux (9) d'un deuxième piston de remplissage (8) ;
b) la canule (13) d'un premier moyen d'application (2) présente une forme externe adaptée à l'espace creux (9) du premier piston de remplissage (8) ; et
c) la canule (13) d'un deuxième moyen d'application (2) présente une forme externe adaptée à l'espace creux (9) du deuxième piston de remplissage (8).

14. Procédé pour la préparation intra-opératoire d'une pâte de substitut osseux et le remplissage de moyens d'application (2) par utilisation du dispositif (1) conformément à l'une des revendications 1 à 10, **caractérisé par** les étapes :
- Remplissage du fond (4) avec la pâte de substitut osseux préparée ;
- Introduction et enfoncement du piston de remplissage (8) dans la cavité (7) du fond (4), de sorte que l'espace creux (9) du piston de remplissage (8) est rempli de pâte de substitut osseux ; et
- Introduction d'une canule (13) d'un moyen d'application (2) dans un des espaces creux (9) du piston de remplissage (8) remplis de pâte de substitut osseux, de sorte que la pâte de substitut osseux présente dans l'espace creux (9) du piston de remplissage (8) est transportée dans la lumière (17) de la canule (13) du moyen d'application (2).

15. Procédé selon la revendication 14, **caractérisé en ce que** préalablement au remplissage du fond l'étape supplémentaire suivante est réalisée :
- Mélange des substances nécessaires à la préparation de la pâte de substitut osseux.
